# EUROPÄISCHE PATENTSCHRIFT

(11) **EP 0 653 218 B1**
(45) Veröffentlichungstag und Bekanntmachung des Hinweises auf die Patenterteilung: **23.09.1998**
(21) Anmeldenummer: 93118465.9
(22) Anmeldetag: 15.11.1993
(51) Int. Cl.: A61M 11/06, B05B 7/00

(54) **Zerstäubervorrichtung**
Atomizer device
Dispositif atomiseur

(43) Veröffentlichungstag der Anmeldung: 17.05.1995
(73) Patentinhaber: PARI GmbH Spezialisten für effektive Inhalation, 82319 Starnberg (DE)
(72) Erfinder: Brugger, Stephan, Dipl.-Wirtsch.-Ing., D-82301 Starnberg (DE)
(74) Vertreter: Ritter und Edler von Fischern, Bernhard, Dipl.-Ing.

(56) Entgegenhaltungen:
- EP-A- 0 170 715
- DE-B- 1 046 264
- DE-C- 3 513 876
- US-A- 2 772 117
- US-A- 3 903 884
- US-A- 4 560 519

## Beschreibung

Die vorliegende Erfindung betrifft eine Zerstäubervorrichtung mit den Merkmalen des Oberbegriffs des Patentanspruchs 1.

Eine derartige Zerstäubervorrichtung ist bekannt aus EP-A-0 170 715 und ist in Fig. 3 dargestellt. Die bekannte Zerstäubervorrichtung besteht aus einem Behälter 36 für das Zerstäubungsgut 16 und einer auf den Behälter aufsetzbaren Verneblerhaube 45, die beide die im wesentlichen rotationssymmetrische Grundform des Gehäuses 30 der Zerstäubervorrichtung festlegen. Durch einen zur Grundform koaxialen Kamin 38 tritt Zuluft in den Zerstäubungsraum ein, wenn der Patient das bereitgestellte Aerosol über den Ansaugstutzen 39 einatmet.

Im Behälter 36 ist mittig ein Düsenkopf 33 angeordnet. Durch dessen Druckgaskanal 32 wird ein gasförmiges Druckmittel geführt und tritt oben aus einer Düsenöffnung aus. Dabei saugt das Druckmittel aus benachbart angeordneten Ansaugkanälen 34 und 35 das Zerstäubungsgut an, da die Düsenöffnungen der Ansaugkanäle 34 und 35 benachbart zur Düsenöffnung des Druckgaskanals 32 angeordnet sind. Nahe der Mündungsebene ist gegenüber der Düsenöffnung des Druckgaskanales 32 ein Gasstromsteuer 37 angeordnet, das auf seiner der Mündungsebene gegenüberliegenden Seite keilförmig ausgebildet ist. Am unteren Ende des Zuluftkamins 38 ist ein zylindrischer Einsatz 43 mit einem sich nach außen und abwärts in den Zerstäubungsraum erstreckenden Prallschirm 44 angeordnet.

Wird über den Anschlußstutzen 31 das gasförmige Druckmittel zugeführt, bilden sich beidseitig des Gasstromsteuers 37 die Aerosolfächer 27 und 28 aus. Ein Teil der Aerosolpartikel der Fächer 27 und 28 prallt auf die Innenseite des Prallschirms 44 und wird dadurch noch weiter zerkleinert. Größere Aerosolpartikel tropfen von der Kante 40 des Prallschirms 44 ab und gelangen so wieder zum Zerstäubungsgut 16 im Behälter 36 zurück.

Um die kurze Bauweise des Zuluftkamins 38 zu ermöglichen, besitzt die bekannte Zerstäubervorrichtung einen im Behälter 36 angeordneten ringförmigen Einsatz 42, wodurch eine Art Labyrinthführung des Aerosolstromes bis zum Ausgangstutzen 39 erzeugt wird. An der Kante 41 des Einsatzes 42 werden weitere größere Partikel aus dem Aerosolstrom ausgefiltert und gelangen in den Behälter 36 zurück. Das so von zu großen Partikeln gefilterte Aerosol verläßt den Zerstäuberkopf durch den Ausgangstutzen 39 und enthält einen hohen Anteil von lungengängigen Aerosolpartikeln, d.h. von Aerosolpartikeln in einem Spektrum von 0,5 bis 5 µm Durchmesser.

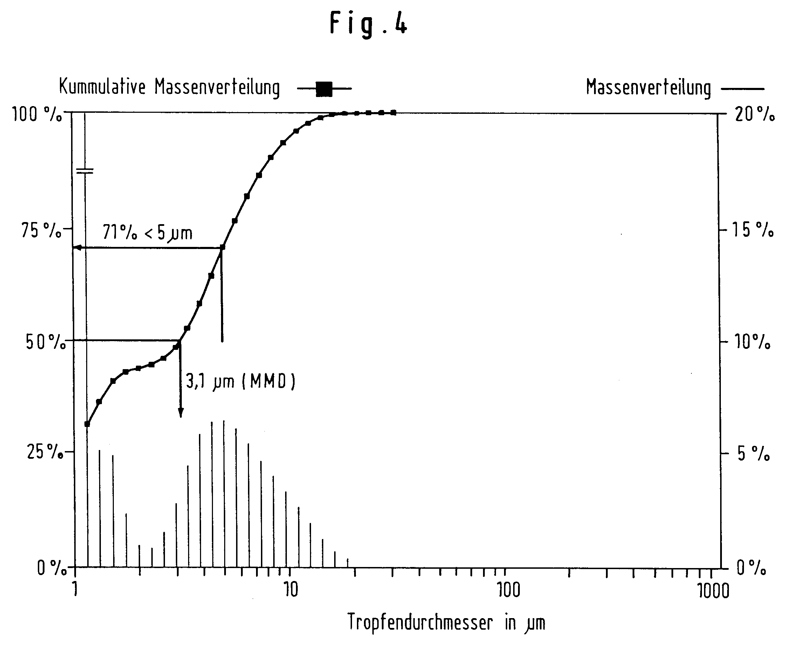

In Fig. 4 ist das Ergebnis einer Messung dargestellt, mit dem das Tröpfchenspektrum der bekannten Zerstäubervorrichtung ermittelt wurde. Bei einem Betriebsdruck von 1,5 bar und einer Gesamtabgabemenge von 0,49 g/min wurde als Zerstäubungsgut eine 0,9%-NaCl-Lösung verwendet. Die durchgesetzte Luftmenge betrug 20 l/min. Wie die in Fig. 4 aufgetragene Meßkurve zeigt, besitzen 71% der erzeugten Aerosoltröpfchen einen Durchmesser von weniger als 5 µm, was gegenüber Zerstäubervorrichtungen ohne Prallschirm oder ohne Gasstromsteuer bereits eine sehr hohe Ausbeute darstellt. Eine weitere Steigerung der Ausbeute war bei Zerstäubervorrichtungen mit nach dem Venturi-Prinzip arbeitenden Düsen nicht zu erwarten.

Davon ausgehend liegt der Erfindung die sich im Bereich der Zerstäuberdüsen stets stellende Aufgabe zugrunde, eine weitere Verbesserung der Ausbeute an lungengängigen Aerosolpartikeln zu erreichen.

Gelöst wird diese Aufgabe mit einer überraschend hohen Steigerung der Ausbeute durch eine Zerstäubervorrichtung mit den Merkmalen des Patentanspruchs 1.

Vorteilhafte Ausgestaltungen ergeben sich aus den Unteransprüchen.

Die Erfindung beruht auf der überraschenden Erkenntnis, daß durch Verlängerung des Prallschirms in den Zerstäubungsraum hinein eine unerwartet hohe Steigerung der Ausbeute an lungengängigen Aerosolpartikeln erreicht werden kann. Zwar konnte erwartet werden, daß durch konstruktive Optimierung der bekannten Zerstäubervorrichtung eine geringfügige Verbesserung des Zerstäubungsvorgangs möglich ist, jedoch konnte eine Steigerung um ca. 20% nicht erwartet werden. Diese überraschend hohe Steigerung des Zerstäubungsergebnisses wird erläutert anhand einer Meßreihe, die unter den oben beschriebenen Bedingungen an Zerstäubern mit dem erfindungsgemäß verlängerten Prallschirm durchgeführt wurden.

Ein Ausführungsbeispiel der Erfindung, das den in der Meßreihe verwendeten Zerstäubern entspricht, wird im folgenden anhand der Figuren genauer beschrieben, in denen zeigt:
- Fig. 1: eine Zerstäubervorrichtung gemäß der vorliegenden Erfindung;
- Fig. 2: ein Diagramm, das das Meßergebnis einer an erfindungsgemäßen Zerstäubern durchgeführten Meßreihe wiedergibt;
- Fig. 3: eine bekannte Zerstäubervorrichtung;
- Fig. 4: ein Diagramm, das das Meßergebnis für die bekannten Zerstäubervorrichtung wiedergibt.

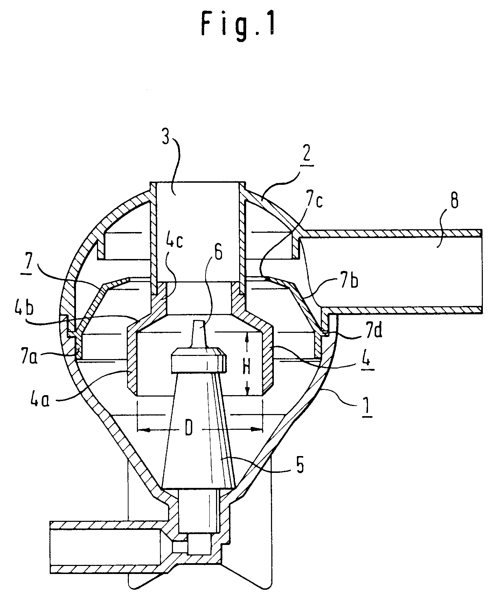

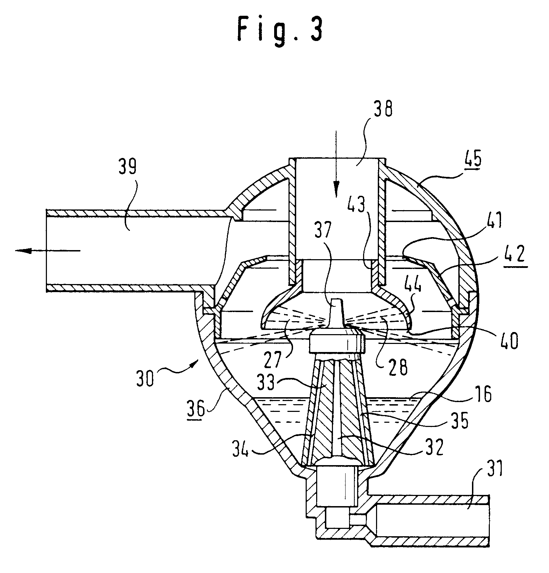

Die in Fig. 1 gezeigte erfindungsgemäße Zerstäubervorrichtung entspricht in ihrem Grundaufbau der eingangs geschilderten Zerstäubervorrichtung aus dem Stand der Technik. Neben einem Behälter 1 für das Zerstäubungsgut ist eine auf den Behälter aufsetzbare Verneblerhaube 2 vorgesehen, die einstückig mit einem Kamin 3 für den Eintritt von Zuluft in den Zerstäubungsraum ausgebildet ist. Der Kamin 3 ist koaxial zur im wesentlichen rotationssymmetrischen Grundform der Zerstäubervorrichtung angeordnet, die sich aus Behälter 1 und Verneblerhaube 2 ergibt. Mittig im Behälter 1 ist eine Zerstäuberdüse 5 mit Gasstromsteuer 6 angeordnet, die beide in Aufbau und Funktion dem Düsenkörper und Gasstromsteuer der eingangs geschilderten, bekannten Zerstäubervorrichtung entsprechend. Ebenso ist bei der erfindungsgemäßen Zerstäubervorrichtung ein Einsatz 7 vorhanden, der in Aufbau und Funktion dem eingangs geschilderten Einsatz bei der bekannten Zerstäubervorrichtung entspricht. Auf die entsprechenden Teile der Beschreibung zum Stand der Technik im Zusammenhang mit Fig. 3 und auf Fig. 3 selbst wird Bezug genommen. Am unteren Ende des Kamins 3 und auf der Höhe der Mündungsebene der Zerstäuberdüse 5 ist der erfindungsgemäß verlängerte Prallschirm 4 angeordnet. Somit verfügt auch die erfindungsgemäße Zerstäubervorrichtung über eine Labyrinthführung für das Aerosol, die durch den Kamin 3, den erfindungsgemäß verlängerten Prallschirm 4 und den Einsatz 7 bestimmt wird. Durch das Labyrinth wird das im Bereich des Gasstromsteuers beidseitig davon erzeugte Aerosol dem Ansaugstutzen 8 zugeführt.

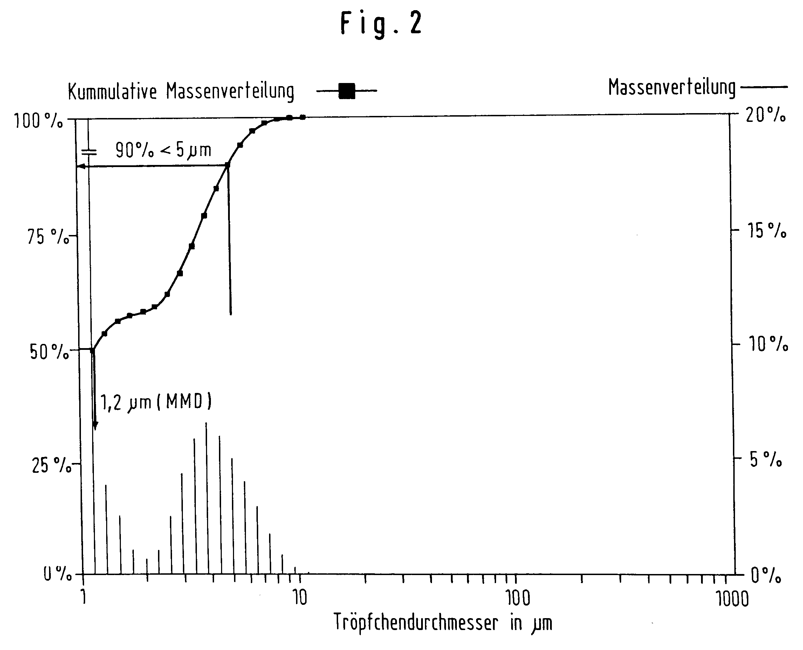

Das von der erfindungsgemäßen Zerstäubervorrichtung bereitgestellte Aerosol besitzt jedoch aufgrund des verlängerten Prallschirms 4 einen erheblich gesteigerten Anteil an lungengängigen Aerosolpartikeln. Diesbezüglich wird auf Fig. 2 verwiesen, die ein Diagramm der Ergebnisse einer Meßreihe zeigt, die an Zerstäubern mit erfindungsgemäß verlängertem Prallschirm durchgeführt wurde. Aus dem Diagramm ist ersichtlich, daß 90% der Aerosolpartikel einen Durchmesser besitzen, der kleiner als 5 µm ist, und damit lungengängig sind. Darüber hinaus kann auch eine Reduzierung des MMD-Wertes auf 1,2 µm festgestellt werden (MMD = medianer Massendurchmesser; der Durchmesser, bei dem die Massenverteilungssumme 50% beträgt, gem. VDI 3491, Blatt 1). Im Vergleich dazu zeigt Fig. 4 einen Wert von 71% bzw. 3,1 µm. Eine abschließende Erklärung für die unerwartet hohe Steigerung an lungengängigen Aerosolpartikeln liegt bislang noch nicht vor; es wird vermutet, daß diese Steigerung auf ein kombinatorisches Zusammenwirken der Labyrinthführung durch den Einsatz 7 und durch den verlängerten Prallschirm 4 zurückzuführen ist. Ferner erscheint eine verbesserte Abscheidewirkung des verlängerten Prallschirms 4 einen Einfluß auf das Zerstäubungsergebnis zu haben.

Beste Ergebnisse wurden erzielt, wenn, wie in Fig. 1 gezeigt, der verlängerte Abschnitt 4a des Prallschirms 4 in Form eines Zylinderringes ausgebildet ist, der über einen kegeligen Abschnitt 4b mit dem wiederum zylindrischen Einsatz 4c verbunden ist. Entscheidend ist auch, daß bestimmte Größenverhältnisse eingehalten werden. So sollte das Verhältnis des Durchmessers D des zylindrischen Verlängerungsteils 4a zur Höhe H dieses Teils im Bereich von 1 bis 4 (D/H = 1 ... 4), vorzugsweise aber im Bereich von 1,25 bis 2,5 (D/H = 1,25 ... 2,5) liegen. Nach den durchgeführten Messungen kann aber davon ausgegangen werden, daß bei anderer Formgebung auch andere Größenverhältnisse zum gewünschten Ergebnis führen.

Besondere Bedeutung besitzt die angefaste Kante am unteren Ende des Verlängerungsteils 4a. Durch die Anfasung wird das Abtropfen zu großer Aerosolpartikel vom Prallschirm 4 verbessert, jedoch bewirkt diese Maßnahme je nach Auslegung des verlängerten Teils 4a nur eine geringe Steigerung des Anteils lungengängiger Aerosolpartikel.

Demgegenüber ist die Auslegung des zylindrischen Einsatzes 7 und die Dimensionierung insbesondere des sich um den Kamin 3 herum ergebenden Ringspaltes von untergeordneter Bedeutung, jedoch nicht ohne Einfluß. Lediglich wenn der Einsatz vollständig weggelassen wird, verschlechtert sich das Ergebnis drastisch. Aus diesem Grund wird auch die kombinatorische Wirkung im Zusammenspiel des verlängerten Prallschirms 4 und des zylindrischen Einsatzes 7 vermutet.

Wie bei der bekannten Zerstäubervorrichtung, besteht der zylindrische Einsatz 7 aus einem Randflansch 7a und einem sich nach oben in den Zerstäubungsraum kegelstumpfförmig verjüngenden Wandteil 7b, dessen Rand unter Ausbildung einer Kante 7c nach innen in Richtung auf den Kamin 3 gezogen ist. Zwischen Randflansch 7a und Wandteil 7b ist eine nach aussen vorspringende Lippe 7d zur Fixierung des Einsatzes an einem entsprechend ausgebildeten Rand des Behälters vorgesehen. Im zusammengebauten Zustand, wie er in Fig. 1 gezeigt ist, verbleibt nur ein Ringspalt zwischen der Außenwand des Kamins 3 und der Kante 7c. Größere Tröpfchen können so auch im Bereich der Kante 7c abgeschieden werden und in den Behälter 1 zurücklaufen.

## Patentansprüche

1. Vorrichtung zum Zerstäuben, Verteilen und Vermischen von flüssigen oder pulverförmigen Stoffen mittels eines Druckgasstromes, insbesondere für Inhalationszwecke, mit
- einem Behälter (1) für das Zerstäubungsgut (16),
- einer Verneblerhaube (2), die auf den Behälter aufsetzbar ist,
- einer Zerstäuberdüse (5), die zentral im Behälter angeordnet ist und bei der das in einem Druckgaskanal geführte Druckmittel das Zerstäubungsgut über Ansaugkanäle ansaugt, deren Düsenöffnungen zur Düsenöffnung des Druckgaskanals benachbart angeordnet sind,
- einem Kamin (3) für den Eintritt von Zuluft in das Innere der Vorrichtung, der koaxial zur Zerstäuberdüse angeordnet ist,
- einem Gasstromsteuer (6), das im Austrittskegel des Druckgases gegenüber der Düsenöffnung des Druckgaskanals und nahe der Mündungsebene angeordnet ist und auf der der Düsenöffnung gegenüberliegenden Seite keilförmig ausgebildet ist,
- einem Einsatz (7) zur Umlenkung des Aerosolstromes, der einen Ringspalt bildend um den Kamin herum angeordnet ist, und
- einem Prallschirm (4), der am unteren Ende des Kamins angeordnet ist und sich in den Zerstäubungsraum erstreckt,
dadurch **gekennzeichnet**, daß
- der Prallschirm (4) einen im wesentlichen rotationssymmetrischen verlängerten Abschnitt (4a) aufweist, der auf Höhe der Mündungsebene der Zerstäuberdüse (5) angeordnet ist und dessen Durchmesser (D) das 1 bis 4-fache von dessen Höhe (H) beträgt.

2. Vorrichtung nach Anspruch 1, dadurch gekennzeichnet, daß der Duchmesser (D) des verlängerten Abschnitts (4a) des Prallschirms (4) das 1,25 bis 2,5-fache von dessen Höhe (H) beträgt.

3. Vorrichtung nach Anspruch 1 oder 2, dadurch gekennzeichnet, daß der verlängerte Abschnitt(4a) des Prallschirms (4) zylindrisch ist.

4. Vorrichtung nach Anspruch 3, dadurch gekennzeichnet, daß der verlängerte Abschnitt (4a) des Prallschirms (4) über einen kegeligen Abschnitt (4b) mit einem zylindrischen Einsatz (4c) verbunden ist, der an den Kamin (3) angepaßt ist.

5. Vorrichtung nach einem der Ansprüche 1 bis 4, dadurch gekennzeichnet, daß der verlängerte Abschnitt (4a) des Prallschirms (4) an seinem in den Zerstäubungsraum ragenden Ende eine Anfasung aufweist.

6. Vorrichtung nach einem der Ansprüche 1 bis 5, dadurch gekennzeichnet, daß der Einsatz (7) rotationssysmmetrisch ist, einen Randflansch, einen sich nach oben in den Zerstäubungsraum kegelstumpfförmig verjüngenden Wandteil, dessen Rand unter Ausbildung einer Kante nach innen in Richtung auf den Kamin gezogen ist, und zwischen Randflansch und Wandteil eine nach außen vorspringende Lippe zur Fixierung des Einsatzes an einem entsprechen ausgebildeten Rand des Behälter und/oder der Verneblerhaube aufweist.

## Claims

1. Device for atomising, distributing and mixing liquid or powdered materials by means of a compressed gas stream, in particular for inhalation purposes, with
- a receptacle (1) for the atomisation product (16),
- a nebuliser hood (2) which can be mounted on the receptacle,
- an atomiser nozzle (5) which is arranged centrally in the receptacle and in which the pressure medium conducted in a compressed gas channel draws in the atomisation product via intake channels whose nozzle openings are arranged adjacent to the nozzle opening of the compressed gas channel,
- a flue (3) for the entry of fresh air into the interior of the device, which is arranged coaxially with the atomiser nozzle,
- a gas stream rudder (6) which is arranged in the outlet cone of the compressed gas opposite the nozzle opening of the compressed gas channel and near the opening plane and is wedge-shaped on the side opposite the nozzle opening,
- an insert (7) for deflecting the aerosol stream, which, forming an annular gap, is arranged around the flue, and
- a baffle screen (4) which is arranged at the lower end of the flue and extends into the atomisation chamber,
characterised in that
- the baffle screen (4) comprises an essentially rotationally symmetrical extended section (4a) which is arranged at the level of the opening plane of the atomiser nozzle (5) and whose diameter (D) is 1 to 4 times the height thereof (H).

2. Device according to claim 1, characterised in that the diameter (D) of the extended section (4a) of the baffle screen (4) is 1.25 to 2.5 times the height thereof (H).

3. Device according to claim 1 or 2, characterised in that the extended section (4a) of the baffle screen (4) is cylindrical.

4. Device according to claim 3, characterised in that the extended section (4a) of the baffle screen (4) is connected by a conical section (4b) to a cylindrical insert (4c) which is adapted to the flue (3).

5. Device according to any of claims 1 to 4, characterised in that the extended section (4a) of the baffle screen (4) comprises a chamfer at its end extending into the atomisation chamber.

6. Device according to any of claims 1 to 5, characterised in that the insert (7) is rotationally symmetrical and comprises an edge flange, a wall portion which tapers frustoconically upwards into the atomisation chamber and whose edge is drawn inwardly in a direction towards the flue, forming a rim, and between edge flange and wall portion an outwardly projecting lip for fixing the insert to a correspondingly shaped edge of the receptacle and/or of the nebuliser hood.

## Revendications

1. Dispositif pour pulvériser, diviser et mélanger des produits liquides ou formant des poudres, à l'aide d'un flux de gaz sous pression, en particulier pour des inhalations comprenant
- un récipient (1) pour le produit à pulvériser (16);
- un couvercle (2) qui est installé sur le récipient de manière amovible;
- une buse (5) de pulvérisation qui est disposée de manière centrale et dans laquelle un milieu sous pression amené par un canal de gaz sous pression aspire le produit à pulvériser par des canaux d'aspiration dont les ouvertures de buse jouxtent les ouvertures de buse du canal de gaz sous pression,
- une cheminée (3) pour l'entrée de l'air d'alimentation à l'intérieur du dispositif, qui est coaxiale par rapport à la buse de pulvérisation,
- un dispositif (6) de commande du gaz sous pression, qui est disposé dans le cône de sortie des gaz sous pression, vis-à-vis l'ouverture de buse du canal de gaz sous pression et près du plan de sortie et qui est réalisé en forme de coin sur le côté opposé à l'ouverture de buse,
- une pièce d'insertion (7) prévue pour dévier le flux de l'aérosol et disposée de manière à former un passage annulaire autour de la cheminée, et
- un écran (4) à impacts qui est disposé sous l'extrémité inférieure de la cheminée et qui fait saillie dans la chambre de pulvérisation,
caractérisé en ce que
- l'écran (4) à impacts comprend un portion allongée (4a) qui présente essentiellement une symétrie de rotation, qui est disposée à la hauteur du plan de sortie de la buse de pulvérisation (5) et dont le diamètre (D) est égal à 1 à 4 fois la hauteur (H).

2. Dispositif selon la revendication 1, caractérisé en ce que le diamètre (D) de la section allongée (4a) de l'écran (4) à impacts est égal à 1,25 à 2,5 fois sa hauteur (H).

3. Dispositif selon la revendication 1 ou 2, caractérisé en ce que la section allongée (4a) de l'écran (4) à impacts est cylindrique.

4. Dispositif selon la revendication 3, caractérisé en ce que la section allongée (4a) de l'écran (4) à impacts est raccordée par une section conique (4b) à un adaptateur cylindrique (4c), qui est adapté à la cheminée.

5. Dispositif selon l'une des revendications 1 à 4, caractérisé en ce que la section allongée (4a) de l'écran (4) à impacts comporte un chanfrein sur son extrémité s'étendant dans la chambre de pulvérisation.

6. Dispositif selon l'une des revendications 1 à 5, caractérisé en ce que la pièce d'insertion (7) présente une symétrie de rotation, qu'elle comprend une bride latérale, une portion de paroi tronconique se rétrécissant vers le haut dans la chambre de pulvérisation et ayant un bord orienté, après un angle, en direction de la cheminée et, entre la bride latérale et la partie de paroi, une lèvre dirigée vers l'extérieur, pour fixer la pièce d'insertion sur un bord correspondant du récipient et/ou du couvercle.
